# EUROPÄISCHE PATENTSCHRIFT

(11) **EP 1 083 902 B1**
(45) Veröffentlichungstag und Bekanntmachung des Hinweises auf die Patenterteilung: **26.11.2003**
(21) Anmeldenummer: 99915690.4
(22) Anmeldetag: 23.03.1999
(51) Int. Cl.: A61K 31/54, A61K 9/20

(54) **NEUE GALENISCHE ZUBEREITUNGSFORMEN VON MELOXICAM ZUR ORALEN APPLIKATION**
NOVEL GALENIC FORMULATIONS OF MELOXICAM FOR ORAL ADMINISTRATION
NOUVELLES FORMES GALENIQUES DE MELOXICAME A ADMINISTRER PAR VOIE ORALE

(30) Priorität: 27.03.1998 EP 98105569
(43) Veröffentlichungstag der Anmeldung: 21.03.2001
(73) Patentinhaber: Boehringer Ingelheim Pharma GmbH & Co.KG, 55218 Ingelheim am Rhein (DE)
(72) Erfinder: BOCK, Thomas, D-88400 Biberach (DE); SÄGMÜLLER, Paul, D-88368 Bergatreute (DE); SIEGER, Peter, D-88441 Mittelbiberach (DE); TÜRCK, Dietrich, D-89077 Ulm (DE)
(74) Vertreter: Laudien, Dieter, Dr.
(86) Internationale Anmeldenummer: EP9901949
(87) Internationale Veröffentlichungsnummer: WO99049867

(56) Entgegenhaltungen:
- EP-A- 0 002 482
- WO-A-97/17978
- WO-A-99/09988
- DE-A- 3 700 172
- LUGER ET AL.: "Structure and physicochemical properties of meloxicam, a new NSAID" EUROPEAN JOURNAL OF PHARMACEUTICAL SCIENCES, Bd. 4, 1996, Seiten 175-187, XP002074736

## Beschreibung

Die Erfindung betrifft neue galenische Zubereitungsformen des zu den NSAID's (nonsteroidal-antiinflammatory drugs) zählenden Wirkstoffs Meloxicam zur oralen Applikation und Verfahren zu ihrer Herstellung.

Die in der Therapie rheumatischer Erkrankungen eingesetzten Arzneistoffe weisen oft sowohl antiphlogistische als auch analgetische Eigenschaften auf. Aus diesem Grund werden sie auch neben der Behandlung chronischer rheumatischer Erkrankungen für akute rheumatische Schübe sowie zur akuten Schmerztherapie eingesetzt.

Viele dieser Arzneistoffe weisen nur eine geringe Löslichkeit auf und werden aus diesem Grunde vom Körper nur langsam absorbiert. Bei der Behandlung akuter Schmerzen ist eine schnelle Wirkstoffanflutung wesentlich, um einen schnellen Wirkungseintritt zu gewährleisten. Es ist deshalb sehr häufig erforderlich, die Lösungsgeschwindigkeit sowie Löslichkeit entsprechender Wirkstoffe heraufzusetzen.

Für bekannte Arzneistoffe in diesem Indikationsgebiet wurden hierbei unterschiedliche Wege beschritten, z.B. wurden Ibuprofen und Diclofenac in Form ihrer Salze oder Piroxicam als β-Cyclodextrin-Einschlußverbindungen eingesetzt. Dennoch zeigen diese Wirkstoffe nach oraler Applikation eine nicht immer für eine schnelle Wirkung ausreichende Plasmakonzentration innerhalb kurzer Zeit. Die pharmakokinetischen Unterschiede von Ibuprofen-Lysinat im Vergleich mit der Ibuprofen-Säure sind z. B. in Int. J. Clin. Pharmacol. , Ther. Toxicol. ,Vol. 27, No. 7, 324 - 328 (1989) beschrieben. Es wird angegeben, daß der mittlere Peak-Plasmaspiegel gemessen an 8 fastenden Probanden im Falle des Ibuprofen-Lysinats (1000 mg, Filmtablette) im Mittel 0,55 h nach Applikation erzielt wurde und 69,1 µg/ml betrug, während die entsprechenden Werte für die Ibuprofen-Säure (600 mg, Zucker-beschichtete Tablette) mit 0,89 h und 50,8 µg/ml angegeben sind. Bei nicht-fastenden Probanden verlieren die Unterschiede nach Angabe der Autoren an statistischer Signifikanz und betragen für Ibuprofen-Lysinat 50,3 µg/ml nach 1,18 h sowie für Ibuprofen-Säure 44,6 µg/ml nach 1,55 h.

In der DE 37 00 172 wird beschrieben, daß viele NSAID's in Wasser schwer löslich sind und deshalb zur Herstellung von parenteralen Formulierungen wenig geeignet sind. Zur Überwindung dieses Problems wird die Verwendung von N-(Methyl)-glucamin- und Glucaminsalzen einer Reihe von NSAID's, unter anderem von Isoxicam, Tenoxicam und Piroxicam vorgeschlagen. Eine parenterale Piroxicam- N-(methyl)-glucamin-Formulierung ist als Beispiel 4 beschrieben. Ferner wird erwähnt, daß diese Salze auch in oralen, rektalen oder topischen Formulierungen verabreicht werden können, die Offenlegungsschrift enthält jedoch keinerlei Daten zur Resorption oraler Formulierungen. Die dort beschriebene Problematik, nämlich die Herstellung einer parenteralen wäßrigen Formulierung eines vergleichsweise schwerlöslichen Wirkstoffs, unterscheidet sich erheblich von der Aufgabenstellung der vorliegenden Erfindung. Diese besteht, wie nachstehend ausgeführt, in der Bereitstellung einer oral applizierbaren, festen Arzneimittelform von Meloxicam, die nach Applikation schnell zu wirksamen Plasmaspiegels führt. Zusätzlich stellte sich der Ausgangspunkt im Fall der vorliegenden Erfindung wesentlich diffiziler dar, da freies Meloxicam Ober einen weiten pH-Bereich etwa um den Faktor 10 weniger wasserlöslich ist als freies Piroxicam (European Journal of Pharmaceutical Science 4 (1996), 175-187, insbesondere Fig. 10 auf Seite 184).

In der DE-A-3700172 werden wasserlösliche Additionssalze von N-(Methyl)-glucamin und Glucamin mit Verbindungen, welche Carboxyl- oder Carbamidreste aufweisen, beschrieben.

Die WO 97/17978 beschreibt Verabreichungsmedien, die Lösungen von Stickstoffmonooxid in Wasser, Alkoholen, Ethem oder Ölen enthalten oder gegebenenfalls essentielle Fettsäuren oder deren C₁₋₈-Alkylester, was die Wirkung analgetischer, antiinflammatorischer und antipyretischer Wirkstoffe verstärkt.

Meloxicam (4-Hydroxy-2-methyl-N-(5-methyl-2-thiazolyl)-2H-1,2-benzothiazin-3-carboxamid-1,1-dioxid) ist ein neuartiges Antirheumaticum, das sich durch seine gute Magenverträglichkeit bei den für eine Therapie notwendigen Dosierungen auszeichnet. Der Wirkstoff und dessen Natrium- sowie N-Methyl-D-glucaminsalz (Megluminsalz) sind in der EP-A-0 002 482 beschrieben. Die entzündungshemmenden sowie schmerzstillenden Eigenschaften von Meloxicam machen diesen Wirkstoff auch für einen Einsatz bei einer Schmerztherapie sehr interessant. Der Wirkstoff besitzt jedoch nur eine geringe Löslichkeit in dem sauren Bereich, der im oberen Teil des Magen-Darm-Kanals vorherrscht. Seine Absorption findet daher mit einer zeitlichen Verzögerung zur Einnahme statt. Maximale Plasmaspiegel werden je nach Formulierung innerhalb von 2 - 8 Stunden erzielt. Die Wirkdauer ist jedoch langanhaltend und die Wirksamkeit sehr hoch. In aller Regel ist daher eine einmal tägliche Dosierung ausreichend. Um diesen auch für eine Schmerztherapie interessanten Wirkstoff zur Behandlung akuter Zustände zu erschließen, ist es erforderlich, eine schnelle Absorption und damit verbunden, einen schnellen Wirkungseintritt zu gewährleisten.

Aufgabe der vorliegenden Erfindung war die Bereitstellung einer oral applizierbaren, festen Arzneimittelform von Meloxicam, aus der der Wirkstoff schnell freigesetzt und absorbiert wird, sodaß die Erzielung eines zur Behandlung akuter Schmerzzustände geeigneten Plasmaspiegels mit ausreichender Schnelligkeit gewährleistet ist. Folgendes Anforderungsprofil kann in diesem Zusammenhang definiert werden:

Der maximale Plasmaspiegel Cₘₐₓ sollte höher sein als nach Applikation einer konventionellen dosisgleichen Meloxicam-Kapselformulierung und sehr viel früher erreicht werden. Ein ausreichend hoher, wirksamer Plasmaspiegel sollte dann über eine gewisse Zeit gehalten werden. Insbesondere sollte Cₘₐₓ spätestens zwei Stunden nach Applikation einer Einzeldosis erreicht werden und bei einer Dosierung von 7,5 mg im Bereich zwischen 650 und 1000 ng/ml liegen. Idealerweise sollte Cₘₐₓ bei dieser Dosierung in etwa dem doppelten maximalen Plasmaspiegel entsprechen, der mit der konventionellen 7.5 mg Kapselformulierung erreicht wird und demgemäß im Bereich zwischen 800 und 900 ng/mL liegen. Nach Überschreiten des maximalen Plasmaspiegels sollte ein Plasmaspiegel von 500 bis 700 ng/ml für 1 bis 3 Stunden gehalten werden, idealerweise jedoch ein Plasmaspiegel von 550 bis 650 ng/ml, der den steady state Durchschnitts-Plasmaspiegeln von etwa 600 ng/ml nach Applikation der konventionellen 7.5 mg Kapseln entspricht. Ferner sollte die Gesamtresorption der erfindungsgemäßen Formulierung und der konventionellen, dosisgleichen Kapselformulierung äquivalent sein.

Meloxicam ist in der Lage, mit anorganischen Basen Salze zu bilden, beispielsweise das Natrium-, Kalium- oder Ammoniumsalz, ebenso wie mit organischen Basen, beispielsweise das Megluminsalz, das Tris-Salz (Tris-(hydroxymethyl)aminomethan) oder Salze mit basischen Aminosäuren wie L-Lysin oder L-Arginin. Im Zusammenhang mit der vorliegenden Problemstellung sind die Löslichkeiten des Wirkstoffs und seiner Salze von Interesse.

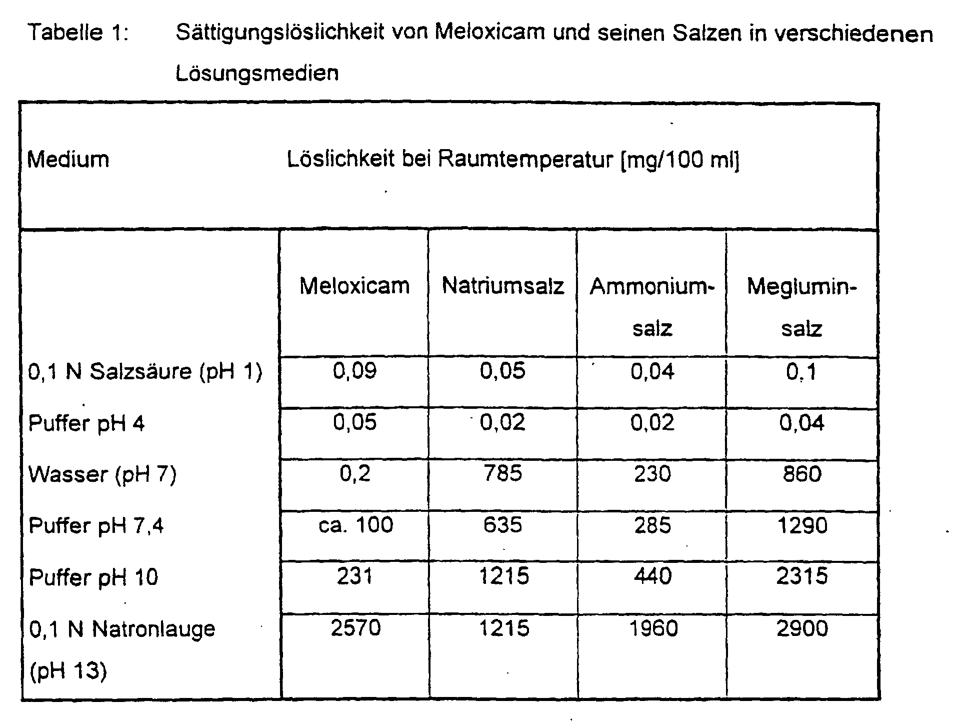

Aus den Daten der Tabelle 1 läßt sich folgendes feststellen:
Sowohl Meloxicam als auch Meloxicamsalze sind in wäßrigen Systemen bei pH-Werten ≤ 4 nur sehr gering löslich, wobei sich keine signifikanten Unterschiede in der Löslichkeit der verschiedenen Verbindungen feststellen lassen. Mit steigendem pH-Wert zwischen 4 und 10 steigt die Löslichkeit der Meloxicamsalze, insbesondere des Natrium- und Megluminsalzes, signifikant stärker an als diejenige des freien Meloxicams, bei sehr hohen pH-Werten nivelliert sich der Effekt der Löslichkeitszunahme. Das freie Meloxicam zeigt eine wesentliche Erhöhung der Löslichkeit erst bei pH-Werten oberhalb 7. Bei pH 13_weisen Meloxicam und seine Salze keine wesentlichen Löslichkeitsunterschiede mehr auf. Dementsprechend können für Meloxicamsalze bei pH-Werten oberhalb 4, beim freien Meloxicam erst bei pH-Werten oberhalb 7 prinzipiell erhöhte Lösungsgeschwindigkeiten erwartet werden.

Es ist bekannt, daß der pH-Wert des Magensaftes, insbesondere bei nüchternen Patienten, zwischen 1 und 6 schwanken kann, bei nicht nüchternen Patienten liegt er meist zwischen 3 und 5.

Da Meloxicamsalze mit Basen in dem im Magen vorherrschenden sauren pH-Bereich eine sehr geringe Löslichkeit aufweisen, wäre zu erwarten, daß ein festes Meloxicam-Salz in dieser Umgebung nur sehr langsam in Lösung und damit zur Resorption gebracht werden kann oder ein bereits gelöstes entsprechendes Meloxicam-Salz in dieser Umgebung ausfällt. Ein wesentlicher Unterschied im Resorptionsverhalten wäre zwischen Meloxicam und seinen Salzen unter diesen Bedingungen aufgrund der Löslichkeitsdaten nicht zu erwarten. Dagegen wäre zu erwarten, daß Salze von Meloxicam mit Basen im weniger sauren Milieu des Dünndarms im Vergleich zum freien Meloxicam beschleunigt und in erhöhtem Maße in Lösung gehen und dort entsprechend schneller absorbiert werden als das freie Meloxicam. Die Freisetzung und Resorption des Wirkstoffs erst im Dünndarm, wobei der Wirkstoff während der Passage durch den Magen durch einen magensaftresistenten Überzug geschützt werden könnte, ist jedoch zur Lösung der vorliegenden Aufgabe ungeeignet. Die Passage durch den Magen nach Einnahme einer entsprechenden Arzneimittelform nimmt zuviel Zeit in Anspruch, sodaß eine wirksame Bekämpfung von Akutschmerz nicht mit befriedigender Schnelligkeit erfolgen kann. Ferner wäre der Zeitverlauf des Wirkungseintritts stark abhängig von der aufgenommenen Nahrung und damit individuellen Schwankungen unterworfen.

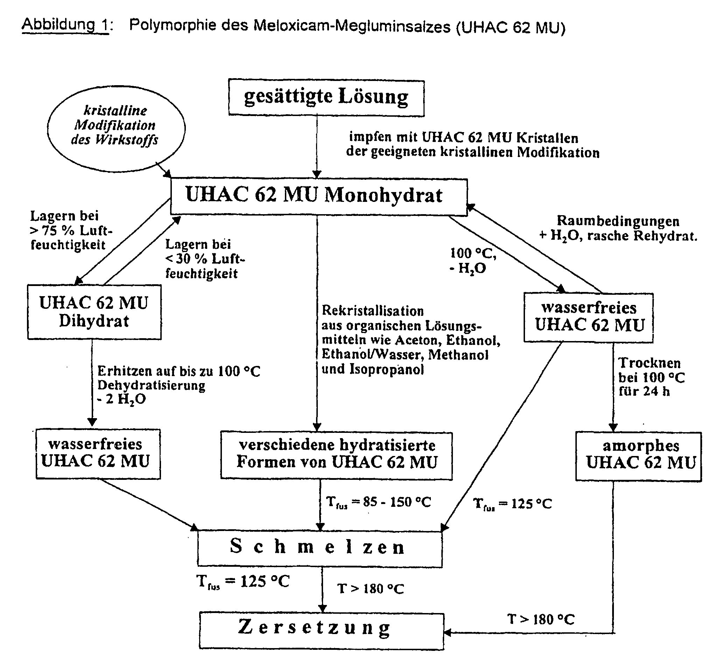

Bei der Auswahl einer geeigneten Wirkstoff-Form zur Entwicklung einer zur Lösung der gestellten Aufgabe geeigneten Formulierung sind neben den pH-abhängigen Löslichkeiten auch weitere physikochemische Eigenschaften von Meloxicam und seiner Salze zu berücksichtigen. Polymorphie der aktiven Komponente, etwa das Vorliegen verschiedener kristalliner, unterschiedlich solvatisierter oder amorpher Modifikationen, kann die chemischen, biologischen und pharmazeutischen Eigenschaften eines Arzneimittels wesentlich beeinflussen. Das Meloxicam-Megluminsalz zeigt eine hohe Tendenz, verschiedene polymorphe Formen zu bilden und kristallisiert aus verschiedenen organischen Lösungsmitteln, beispielsweise aus Aceton, Methanol, Ethanol, Ethanol/Wasser (8:2, v/v) und Isopropanol, in verschiedenen kristallinen Modifikationen, die 4-5% Hydratwasser enthalten, wie sich durch mikroskopische, IR-spektroskopische und thermische Analyse sowie Röntgen-Pulver-Diffraktometrie zeigen läßt. Eine Übersicht über die vorliegende Polymorphie zeigt Abbildung 1. Ferner zeigt das Meloxicam-Megluminsalz nur eine geringe Tendenz zur spontanen Kristallisation.

Die kristalline Monohydrat-Modifikation des Meloxicam-Megluminsalzes ist hygroskopisch, während das Meloxicam-Natriumsalz keine hygroskopischen Eigenschaften aufweist. Unter Raumbedingungen ist das Monohydrat des Meloxicam-Megluminsalzes die stabile Modifikation, wobei jedoch bei einer relativen Luftfeuchtigkeit von über 75% ein Dihydrat gebildet wird. Aus dem Dihydrat kann das eingeschlossene Wasser nur unter Bedingungen sehr hoher Trockenheit entfernt werden. Nach Dehydratisierung wird jedoch keine stabile wasserfreie Modifikation erhalten, sondern die wasserfreie Form absorbiert sehr rasch Wasser unter Bildung der bei Raumbedingungen stabilen Monohydratform. Das Wasser-Sorptions/Desorptionsverhalten von Meloxicam-Meglumin zeigt einen Hysteresis-Effekt. Durch intensives Trocknen über einen längeren Zeitraum geht die wasserfreie Form mehr und mehr in eine amorphe Form über, nach 24 h bei 100 °C ist das Material völlig amorph.

Insbesondere die Polymorphie und Hygroskopie des Meloxicam-Megluminsalzes ließen erhebliche Schwierigkeiten für die Verwendung dieser Form des Wirkstoffs in einer pharmazeutischen Formulierung erwarten, da nur eine reproduzierbar herstellbare, einheitliche und stabile Modifikation eingesetzt werden kann.

Das gemäß Beispiel 3 der EP-A-0 002 482 primär anfallende Meloxicam-Megluminsalz ist wasserfrei und amorph (Trocknung bei 80° über Phosphorpentoxid). Diese Modifikation ist zwar zur Herstellung parenteraler Formulierungen, nicht jedoch zur Herstellung fester Arzneimittelformen geeignet, da diese Form die vorstehend angegebenen Kriterien nicht erfüllt, sondern bei Lagerung unter normalen Raumbedingungen in eine hydratisierte Form übergeht.

Überraschenderweise hat sich gezeigt, daß Meloxicam aus den mit Basen gebildeten Salzen trotz der geringen Löslichkeit bei niedrigen pH-Werten, die dem im Magen vorliegenden Milieu entsprechen, nach der Einnahme wesentlich schneller und in größeren Mengen für eine Absorption zur Verfügung steht als das neutrale Meloxicam. Das Ansteigen der Plamaspiegel nach oraler Applikation der Salze des Meloxicams erfolgt wesentlich schneller als bei Verwendung des reinen Meloxicams. Das hohe Ausmaß und die Schnelligkeit des durch Meloxicamsalze, insbesondere durch das Meloxicam-Megluminsalz, erzielbaren Plasmaspiegelanstiegs konnten unter Berücksichtigung der Eigenschaften der aus dem Stand der Technik bekannten NSAID-Salze vom Fachmann nicht erwartet werden. Der Effekt der Löslichkeitssteigerung durch Verwendung eines Meloxicam-Salzes erfolgt in vivo überraschenderweise auch bei erniedrigten pH-Werten. So wird ermöglicht, daß auch unmittelbar nach Applikation schon große Mengen des Wirkstoffes gelöst werden und damit für eine Aufnahme durch den Körper zur Verfügung stehen.

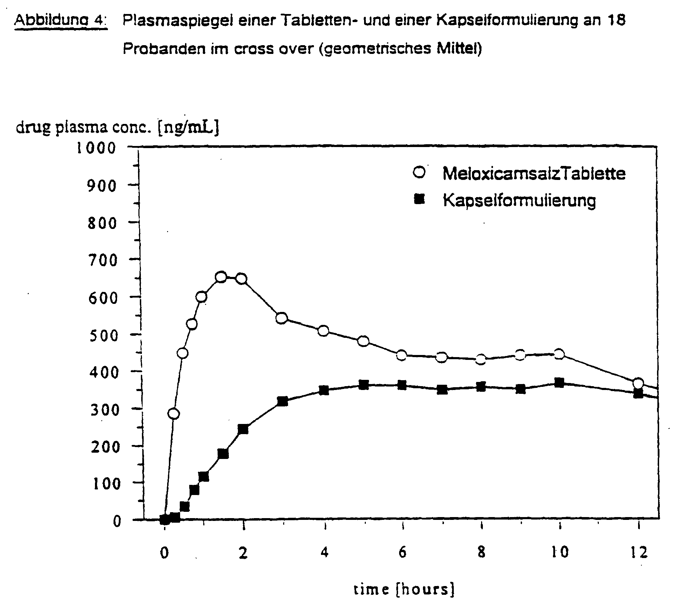

Aus Beispiel 7 zusammen mit Abbildung 4 ist ersichtlich, daß nach peroraler Applikation einer erfindungsgemäßen Meloxicamsalz-Formulierung der Plasmaspiegel erheblich schneller ansteigt, als nach Applikation einer konventionellen Kapselformulierung des neutralen Wirkstoffs. Bereits 15 min nach Applikation der erfindungsgemäßen Meloxicam-Megluminsalz-Formulierung wird ein Plasmaspiegel von 286 ng/ml erzielt, welcher schon nahezu den minimalen Plasmakonzentrationen im steady-state entspricht, während auch 30 min nach Applikation der Vergleichsformulierung noch kein nennenswerter Plasmaspiegel (42 ng/ml) nachgewiesen werden kann. Ferner wird durch die erfindungsgemäße Formulierung nach knapp 2 Stunden ein maximaler Plasmaspiegel von 812 ng/ml erreicht, der etwa doppelt so hoch ist, als der mit der Vergleichsformulierung erzielte minimale steady-state Plasmaspiegel (der maximale Plasmaspiegel wurde aufgrund der zeitlichen Variabilität nicht aus der Mittelwertskurve der Abbildung 4, sondern aus den zugrundeliegenden individuellen Kurven bestimmt). Es kann demnach sowohl ein schneller Wirkungseintritt als auch eine besonders hohe Wirksamkeit in den ersten 2-3 Stunden nach Einnahme einer erfindungsgemäßen Formulierung, insbesondere einer Meloxicam-Megluminsalz-Formulierung, erwartet werden, was für die Bekämpfung akuter Schmerzzustände von Bedeutung ist. Mit der Vergleichsformulierung wird dagegen kein ausgeprägter Plasmaspiegel-Peak erzielt, sondern der Plasmaspiegel steigt mehr oder weniger kontinuierlich bis zum Erreichen eines Plateaus im steady state an.

Die AUC_{0-∞} (AUC: area under the plasma concentration-time curve, _{0-∞}: vom Zeitpunkt 0 der Applikation bis unendlich; Maß für die Resorption) der konventionellen Kapselformulierung gemäß Abbildung 4 beträgt 14.1 µg h/mL, diejenige der erfindungsgemäßen Meloxicam-Megluminsalz-Formulierung 15.0 µg h/mL, beide sind bezüglich dieses Parameters als äquivalent zu betrachten.

Andere Ansatzpunkte zur Lösung der Aufgabe der vorliegenden Erfindung, etwa die Bildung von Einschlußverbindungen von Meloxicam mit β-Cyclodextrin, erbrachten keine ausreichend hohen Plasmakonzentrationen innerhalb kurzer Zeit. Ebenso war die Verpressung eines Gemisches der beiden Einzelkomponenten Meloxicam und Meglumin nicht zur Lösung der Aufgabe der vorliegenden Erfindung geeignet.

Gegenstand der Erfindung ist demnach die Verwendung eines Meloxicamsalzes einer anorganischen oder organischen Base zur Herstellung einer oral applizierbaren, festen Arzneimittelform, aus der der Wirkstoff schnell freigesetzt und absorbiert wird, zur Schmerztherapie, insbesondere zur Behandlung akuter rheumatischer Schübe sowie zur Bekämpfung des Akutschmerzes. Hierbei kommen beispielsweise das Natrium-, Kalium- oder Ammoniumsalz, das Megluminsalz, das Tris-Salz oder das Salz einer basischen Aminosäure wie L-Lysin oder L-Arginin in Frage. Bevorzugt sind das Meloxicam-Megluminsalz und das Meloxicam-Natriumsalz, besonders bevorzugt ist das Meloxicam-Megluminsalz, beispielsweise das Meloxicam-Megluminsalz Dihydrat oder insbesondere das Meloxicam-Megluminsalz Monohydrat.

Um nach peroraler Applikation eine schnelle Wirkstofffreisetzung sicherzustellen, ist es weiterhin vorteilhaft, wenn die hergestellte Arzneiform eine sehr kurze Zerfallzeit aufweist, da in der Regel erst nach dem Zerfall eine Wirkstofffreisetzung in verstärktem Maße stattfinden kann. Es hat sich herausgestellt, daß eine ausreichend kurze Zerfallszeit auch dann erreicht werden kann, wenn der Wirkstoff zusammen mit geeigneten Hilfsstoffen wie Laktose, Dicalciumphosphat, Cellulose sowie geeigneten Zerfallhilfsmitteln wie quervernetztes Polyvinylpyrrolidon oder Natriumstärke direkttablettiert wird, also die entsprechenden Pulvermischungen ohne eine sonst weitgehend übliche Granulation des Pulvers vor der Verpressung direkt zu Tabletten verpreßt werden. Dies bietet den Vorteil eines vereinfachten und billigeren Herstellverfahrens.

Ein zweiter Gegenstand der Erfindung ist demnach eine oral applizierbare, feste Arzneimittelform von Meloxicam, aus der der Wirkstoff schnell freigesetzt und absorbiert wird, zur Schmerztherapie, insbesondere zur Behandlung akuter rheumatischer Schübe sowie zur Bekämpfung des Akutschmerzes, dadurch gekennzeichnet, daß Meloxicam in Form eines Salzes mit einer anorganischen oder organischen Base, gegebenenfalls zusammen mit üblichen Hilfsstoffen oder/und Trägerstoffen, in einer schnell zerfallenden, durch Direkttablettierung hergestellten Tablette vorliegt.

Geeignete Salze mit einer anorganischen Base sind beispielsweise das Natrium-, Kaliumoder Ammoniumsalz des Meloxicams. Als Salze mit organischen Basen kommen beispielsweise das Megluminsalz, das Tris-Salz oder ein Salz des Meloxicams mit einer basischen Aminosäure wie L-Lysin oder L-Arginin in Betracht. Als besonders vorteilhaft im Sinne der vorliegenden Erfindung haben sich das Meglumin- und das Natriumsalz des Meloxicams erwiesen, wobei das Meloxicam-Megiuminsalz besonders bevorzugt ist, beispielsweise das Meloxicam-Megluminsalz Dihydrat oder insbesondere das Metoxicam-Megluminsalz Monohydrat.

Als Hilfsstoffe oder Trägerstoffe kommen beispielsweise mikrokristalline Cellulose, Laktose, quervernetztes Polyvinylpyrrolidon, Magnesiumstearat, Dicalciumphosphat und verschiedene Stärken in Betracht.

Ein dritter Gegenstand der Erfindung ist ein Verfahren zur Herstellung einer oral applizierbaren, festen Arzneimittelform von Meloxicam, die eine kurze Zerfallszeit besitzt und aus der der Wirkstoff schnell freigesetzt und absorbiert wird, zur Schmerztherapie, insbesondere zur Behandlung akuter rheumatischer Schübe sowie zur Bekämpfung des Akutschmerzes, dadurch gekennzeichnet, daß ein gegebenenfalls pulverisiertes Meloxicamsalz einer anorganischen oder organischen Base mit geeigneten pulverisierten Hilfsstoffen und / oder Trägerstoffen innig vermischt und ohne Granulation des Pulvers vor der Verpressung direkt zu Tabletten verpreßt wird. Es kommen hierbei beispielsweise die vorstehend genannten Meloxicamsalze in Frage, wobei das Meglumin- und das Natriumsalz des Meloxicams bevorzugt sind. Besonders bevorzugt ist das Meloxicam-Megluminsalz, beispielsweise das Meloxicam-Megluminsalz Dihydrat oder insbesondere das Meloxicam-Megluminsalz Monohydrat.

Wie eingangs erwähnt, ließen insbesondere die Polymorphie und Hygroskopie des Meloxicam-Megluminsalzes erhebliche Schwierigkeiten für die Verwendung des Wirkstoffs in dieser Form zur Lösung der Aufgabe der Erfindung erwarten, da in einer pharmazeutischen Formulierung nur eine reproduzierbar herstellbare, einheitliche und stabile Modifikation einsetzbar ist. Überraschenderweise kann diese Bedingung mit dem Meloxicam-Megluminsalz erfüllt werden, wenn bei der Kristallisation des Salzes aus einer Mischung aus einem mit Wasser mischbaren organischen Lösungsmittel und Wasser der Mischung Impfkristalle bestehend aus kristallinem Meloxicam-Megluminsalz Monohydrat, vorzugsweise Impfkristalle einer zuvor aus Aceton/Wasser kristallisierten Meloxicam-Megluminsalz Monohydrat-Form, zugesetzt werden. Es wird dann reproduzierbar und einheitlich ein Produkt übereinstimmend mit der Kristallform der eingesetzten Impfkristalle erhalten.

Aus dem so hergestellten kristallinen Meloxicam-Megluminsalz Monohydrat läßt sich durch Konditionieren des Monohydrats bei hoher Luftfeuchtigkeit das kristalline Meloxicam-Megluminsalz Dihydrat erhalten.

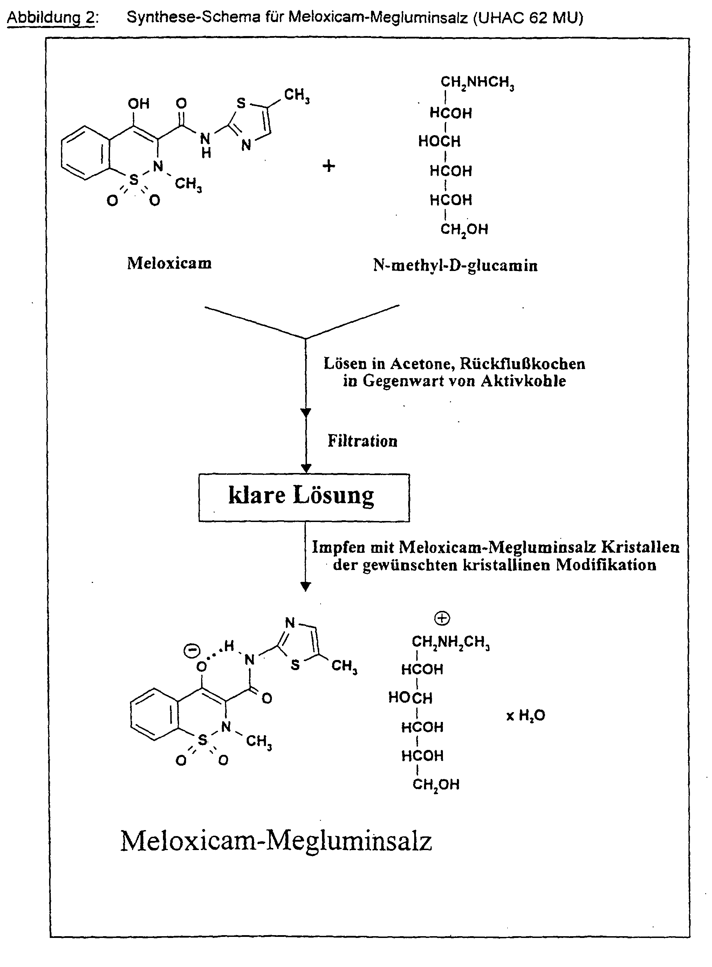

Aufgrund der niedrigen Tendenz zur spontanen Kristallisation und der hohen Tendenz zur Bildung unterschiedlicher polymorpher Formen empfiehlt es sich, im letzten Schritt der Herstellung des festen Meloxicam-Megluminsalzes für die pharmazeutische Verwendung die Lösung mit Kristallen der gewünschten Monohydratform anzuimpfen. Falls gewünscht, läßt sich aus der Monohydratform dann, wie vorstehend erwähnt, die Dihydratform erhalten. Das Syntheseschema ist in Abbildung 2 wiedergegeben.

Ein vierter Gegenstand der Erfindung ist demnach das kristalline Meloxicam-Megluminsalz Monohydrat, ein Verfahren zu dessen Herstellung, in dem Meloxicam und Meglumin in einer Mischung aus einem mit Wasser mischbaren organischen Lösungsmittel und Wasser erhitzt werden und die Mischung zur Kristallisation mit Meloxicam-Megluminsalz Monohydrat-Impfkristallen versetzt wird, und eine oral applizierbare, feste Arzneimittelform, enthaltend Meloxicam in Form des kristallinen Meloxicam-Megluminsalz Monohydrats.

Als organisches Lösungsmittel kommen beispielsweise Aceton, Methanol, Ethanol, n-Propanol, i-Propanol, Tetrahydrofuran oder Dioxan in Frage, vorzugsweise Aceton, Ethanol, Tetrahydrofuran und Dioxan. Besonders bevorzugt sind Aceton und Ethanol, insbesondere jedoch Aceton.

In der Mischung können organisches Lösungsmittel und Wasser im Volumenverhältnis von 10:1 bis 100:1, vorzugsweise in einem Verhältnis von 20:1 bis 50:1 oder besonders bevorzugt in einem Verhältnis von 35:1 bis 45:1 eingesetzt werden, wobei ein Verhältnis von etwa 40:1 bei Verwendung von Aceton besonders geeignet ist.

Meloxicam und Meglumin können beispielsweise in einem molaren Verhältnis von 1:1,5 bis 1,5:1 eingesetzt werden, vorzugsweise in einem molaren Verhältnis von 1:1,2 bis 1,2:1, insbesondere jedoch in äquimolarem Verhältnis.

Zweckmäßigerweise kann die Mischung unter Zusatz von Aktivkohle erhitzt werden, die vor dem Zusatz der Impfkristalle wieder entfernt wird.

Die Menge der zugesetzten Impfkristalle ist abhängig vom verwendeten Lösungsmittelsystem und der Ansatzgröße. Beispielsweise können bei einem Ansatz von A = 12,5 kg Meloxicam der Mischung B = 5 bis 50 g Meloxicam-Megluminsalz Monohydrat-Impfkristalle (Gew.-Verhältnis A : B = 125 : 0,05 - 0,5) zugefügt werden, wobei bei Verwendung des Lösungsmittels Aceton/Wasser ein Zusatz von 5 bis 30 g, insbesondere beim Verhältnis Aceton : Wasser = 40:1 jedoch ein Zusatz an Impfkristallen von 10 bis 15 g besonders geeignet ist. Es ist dem Fachmann ohne weiteres möglich, die geeignete Menge an Impfkristallen bei gegebener Ansatzgröße und gegebenem Lösungsmittelsystem zu ermitteln.

Nach Zusatz der Impfkristalle wird die Mischung auf 10 bis 30 °C abgekühlt, zweckmäßigerweise jedoch auf eine Temperatur von etwa 20 °C. Vorzugsweise wird die Mischung anschließend nochmals bis zum Rückfluß erhitzt und dann langsam auf eine Temperatur zwischen 10 und 30 °C abgekühlt, bevorzugt auf 15 bis 25°C, zweckmäßigerweise jedoch auf etwa 20 °C. Hierbei entsteht eine feinkristalline Kristallsuspension des gewünschten Meloxicam-Megluminsalz Monohydrats, die auf übliche Weise aufgearbeitet wird. Die Pulver X-Ray Reflexe der besonders bevorzugten Meloxicam Megluminsalz Monohydrat-Modifikation sind in der nachstehenden Tabelle 2 enthalten.

Ein fünfter Gegenstand der Erfindung ist das kristalline Meloxicam-Megluminsalz Dihydrat, ein Verfahren zu dessen Herstellung, in dem kristallines Meloxicam-Megluminsalz Monohydrat bei hoher Luftfeuchtigkeit konditioniert wird, und eine oral applizierbare, feste Arzneimittelform, enthaltend Meloxicam in Form des kristallinen Meloxicam-Megluminsalz Dihydrats.

Die Konditionierung wird durch Lagerung während mindestens eines Tages, bevorzugt während mindestens fünf Tagen, bei hoher relativer Luftfeuchtigkeit durchgeführt. Die relative Luftfeuchtigkeit sollte hierbei mindestens 75 %, bevorzugt mindestens 85 %, betragen. Die Pulver X-Ray Reflexe der besonders bevorzugten Meloxicam Megluminsalz Dihydrat-Modifikation sind in der nachstehenden Tabelle 3 enthalten.

Ein sechster Gegenstand der Erfindung ist ein Verfahren zur Herstellung einer oral applizierbaren, festen Arzneimittelform enthaltend Meloxicam in Form des Meloxicam-Megluminsalz Monohydrats, die eine kurze Zerfallszeit besitzt und aus der der Wirkstoff schnell freigesetzt und absorbiert wird, zur Schmerztherapie, insbesondere zur Behandlung akuter rheumatischer Schübe sowie zur Bekämpfung des Akutschmerzes, in dem Meloxicam und Meglumin in einer Mischung aus einem mit Wasser mischbaren organischen Lösungsmittel und Wasser erhitzt werden, die Mischung zur Kristallisation mit Meloxicam-Megluminsalz Monohydrat-Impfkristallen versetzt wird, anschließend auf übliche Weise kristallines Meloxicam-Megluminsalz Monohydrat isoliert und gewünschtenfalls pulverisiert wird und anschließend das Meloxicam-Megluminsalz Monohydrat mit geeigneten pulverisierten Hilfsstoffen und / oder Trägerstoffen innig vermischt und ohne Granulation des Pulvers direkt zu Tabletten verpreßt wird.

Ein siebter Gegenstand der Erfindung ist ein Verfahren zur Herstellung einer oral applizierbaren, festen Arzneimittelform enthaltend Meloxicam in Form des Meloxicam-Megluminsalz Dihydrats, die eine kurze Zerfallszeit besitzt und aus der der Wirkstoff schnell freigesetzt und absorbiert wird, zur Schmerztherapie, insbesondere zur Behandlung akuter rheumatischer Schübe sowie zur Bekämpfung des Akutschmerzes, in dem kristallines Meloxicam-Megluminsalz Monohydrat bei hoher relativer Luftfeuchtigkeit konditioniert wird, das so erhaltene Meloxicam-Megluminsalz Dihydrat gewünschtenfalls pulverisiert und anschließend mit geeigneten pulverisierten Hilfsstoffen und / oder Trägerstoffen innig vermischt und ohne Granulation direkt zu Tabletten verpreßt wird.

Die folgenden Beispiele sollen die Erfindung näher erläutern:

### Beispiel 1: Meloxicam Megluminsalz Monohydrat

12,5 kg (35,57 Mol) Meloxicam und 6,9 kg (35,57 Mol) Meglumin werden unter Rühren nacheinander in eine Mischung aus 275 I Aceton und 7 I Wasser in einem geeigneten Reaktor I eingetragen, anschließend wird 1 kg technische Aktivkohle zugegeben. Die Reaktionsmischung wird erhitzt und 30 Minuten am Rückfluß gekocht. Danach wird die Mischung über ein Druckfilter in einen zweiten Reaktor II abgedrückt. Reaktor I und der Druckfilter werden mit 10 I Aceton nachgewaschen. Die Mischung wird mit 10 -15 g Meloxicam Megluminsalz Monohydrat-Impfkristallen versetzt, auf 20 °C gekühlt und 2 Stunden bei dieser Temperatur gerührt. Anschließend wird die Mischung erhitzt, 15 Minuten am Rückfluß gekocht und dann langsam auf 20°C abgekühlt, wobei eine feinkristalline Kristallsuspension entsteht. Diese wird 15 Stunden bei 20°C gerührt. Danach wird die Kristallsuspension über eine Zentrifuge abgeschleudert und anschließend trockengeschleudert. Der Schleuderkuchen wird mit 35 I Aceton gewaschen und erneut trockengeschleudert. Das Produkt wird im Trockenschrank bei 20 - 35 °C mit Frischluft während etwa 24 Stunden getrocknet. Ausbeute: 90,1 % d. Th.; schwach gelbes, kristallines Pulver, nadelförmige Kristalle; Schmelzpunkt: 120 °C.

Das so erhaltene kristalline Meloxicam Megluminsalz Monohydrat wurde IR-spektroskopisch, mittels Röntgen-Pulver Diffraktion (X-Ray Powder Diffraction) und mittels thermischer Analyse (Thermogravimetrie = TG; Differential Scanning Calorimetry = DSC) untersucht.

### 1.1 IR Spektroskopie

| | |
|---|---|
| Gerät | Nicolet FTIR Spectrometer Magna - IR 550 |
| Software | Nicolet Software Packet OMNIC, Version 1.20 |
| Technik | Transmittance, KBr-Pellets (2.5 µmol Substanz/300 mg KBr), |
| | N₂-Spülung (Fluß: 15 I N₂/min) |

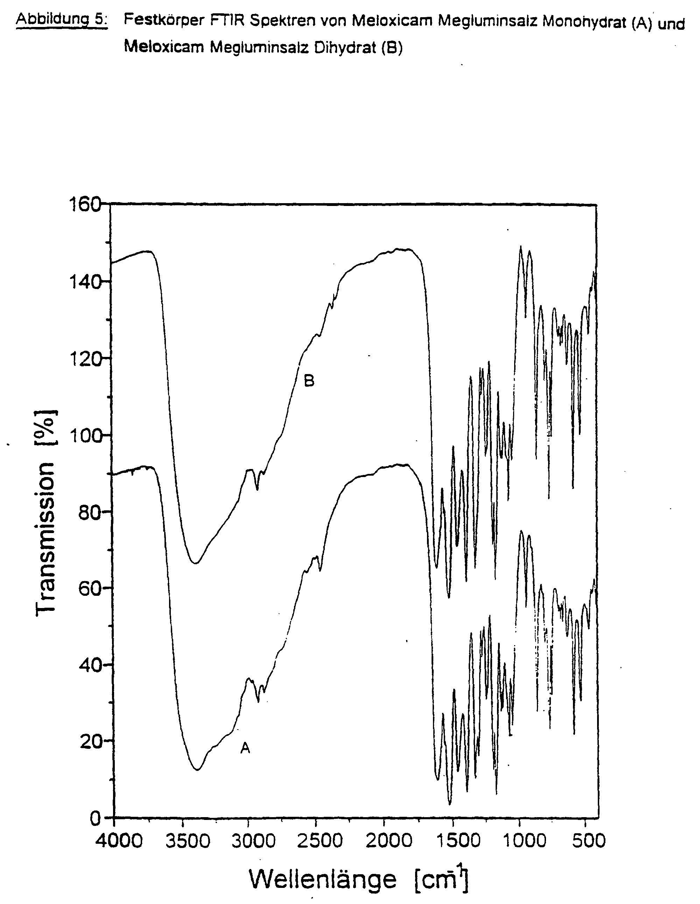

Das FTIR-Spektrum ist in Abbildung 5 wiedergegeben.
Im Vergleich mit dem FTIR-Spektrum der Dihydratform zeigt sich ein signifikanter Unterschied in der aufgespaltenen Bande bei etwa 1300 cm⁻¹ im Spektrum der Monohydratform, ansonsten sind die Spektren sehr ähnlich.

### 1.2 Röntgen-Pulver Diffraktion

| | |
|---|---|
| Gerät | Philips X-Ray Pulver Diffractometer, |
| | CuK_{α} - Strahlung, α = 1.5418 Å, 35 mA, 35 kV |
| Software | Software Paket GUFI 4.06 zur Daten Interpretation, |
| | Software Paket ORIGIN zur Daten Präsentation |
| Parameter | Range: 3 - 50 ° 2Θ Step |
| | scan: 0.01 ° 2Θ Schrittweite, 2 sec Zählzeit für jeden Schritt |

**Tabelle 2:**

| Pulver X-Ray Reflexe und deren Intensitäten (normalisiert), Meloxicam Megluminsalz Monohydrat | | |
|---|---|---|
| **2Θ**_{**exp**} **[°]** | **d**_{**exp**} **[Å]** | **Intensität I/I**_{**o**} |
| 6.50 | 13.6 | 32 |
| 11.26 | 7.85 | 9 |
| 13.03 | 6.79 | 78 |
| 13.42 | 6.59 | 61 |
| 14.92 | 5.93 | 90 |
| 15.91 | 5.57 | 10 |
| 16.66 | 5.32 | 7 |
| 17.84 | 4.97 | 20 |
| 18.38 | 4.82 | 20 |
| 18.58 | 4.77 | 47 |
| 19.24 | 4.61 | 25 |
| 20.29 | 4.37 | 5 |
| 20.47 | 4.34 | 16 |
| 21.97 | 4.04 | 13 |
| 22.72 | 3.91 | 3 |
| 23.18 | 3.84 | 7 |
| 23.34 | 3.81 | 4 |
| 23.49 | 3.78 | 4 |
| 23.79 | 3.74 | 8 |
| 23.97 | 3.71 | 6 |
| 25.45 | 3.50 | 13 |
| 25.83 | 3.45 | 100 |
| 26.30 | 3.39 | 14 |
| 26.95 | 3.31 | 6 |
| 27.25 | 3.27 | 4 |
| 27.89 | 3.20 | 3 |
| 28.55 | 3.12 | 3 |
| 29.09 | 3.07 | 7 |
| 29.53 | 3.02 | 10 |
| 30.18 | 2.96 | 8 |
| 31.19 | 2.87 | 4 |
| 36.01 | 2.49 | 9 |
| 36.16 | 2.48 | 8 |
| 37.73 | 2.38 | 8 |
| 38.64 | 2.33 | 6 |
| 39.78 | 2.26 | 8 |

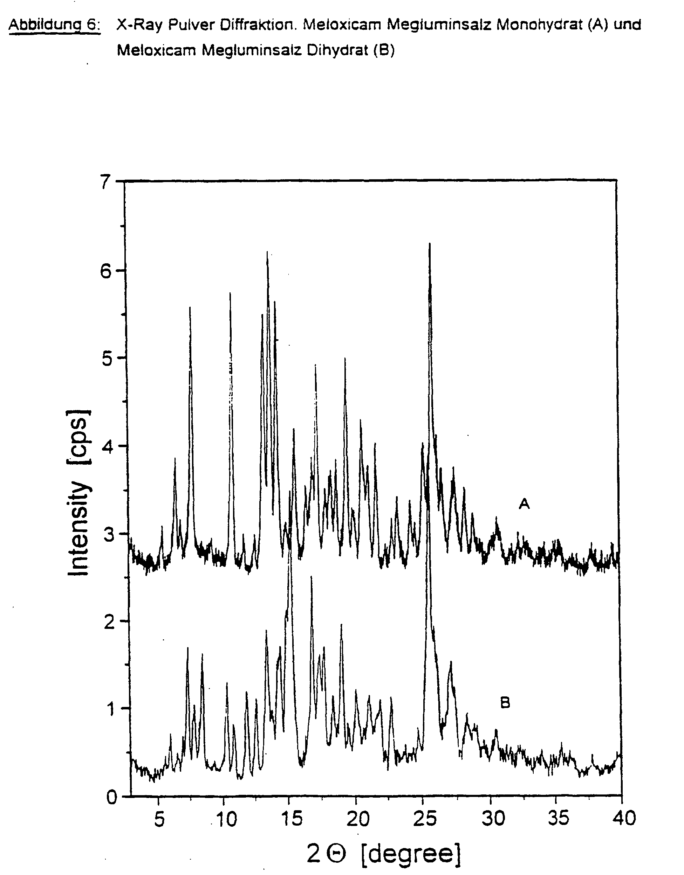

Das Röntgen-Pulver Diffraktions-Pattern ist in Abbildung 6 wiedergegeben.

### 1.3 Thermische Analyse

| | | |
|---|---|---|
| TG | Gerät | Mettler Microbalance M3, Temperature-controller TC15 |
| | Software | Mettler Software Paket STAR |
| | Technik | γ-Al₂O₃ Schmelztiegel, Heizrate: 10 K/min, N₂-Atmosphäre |
| | | |
| | DSC: Gerät | Mettler DSC-20, temperature controller TC15 |
| | Software | Mettler Software Paket STAR |
| | Technik | offene Al Schmelztiegel, Heizrate: 3 und 10 K/min, N₂-Atmosphäre |

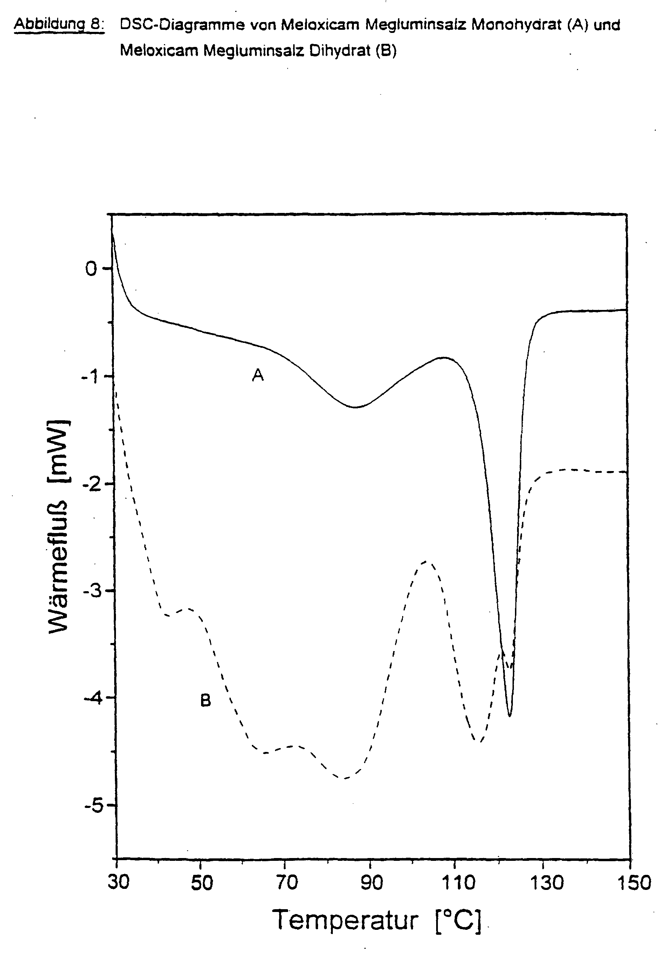

Es kann eine klare Korrelation der im DSC-Diagramm beobachteten endothermen Peaks mit Dehydratisierungs- bzw. Schmelzprozessen festgestellt werden. Dehydratisierung und Schmelzen sind klar getrennte Prozesse.
Das DSC-Diagramm ist in Abbildung 8 wiedergegeben.

### Beispiel 2: Meloxicam Megluminsalz Dihydrat

Kristallines Meloxicam Megluminsalz Dihydrat wird durch Lagerung des gemäß Beispiel 1 erhaltenen kristallinen Meloxicam Megluminsalz Monohydrats während fünf Tagen über gesättigter Kaliumchloridlösung bei einer relativen Luftfeuchtigkeit von 86 % und einer Temperatur von 20 °C erhalten.

Das so erhaltene kristalline Meloxicam Megluminsalz Dihydrat wurde IR-spektroskopisch, mittels Röntgen-Pulver Diffraktion (X-Ray Powder Diffraction) und mittels thermischer Analyse (Thermogravimetrie = TG; Differential Scanning Calorimetry = DSC) untersucht. Es wurden die in Beispiel 1 genannten Geräte, Software und Parameter verwendet.

### 2.1 IR Spektroskopie: Das FTIR-Spektrum ist in Abbildung 5 wiedergegeben.

### 2.2 Röntgen-Pulver Diffraktion:

**Tabelle 3:**

| Pulver X-Ray Reflexe und deren Intensitäten (normalisiert), Meloxicam Megluminsalz Dihydrat | | |
|---|---|---|
| **2 Θ [°]** | **d**_{**exp**} **[Å]** | **I/I**_{**O**} **[%]** |
| 5,99 | 14,8 | 13 |
| 6,95 | 12,7 | 13 |
| 7,36 | 12, 0 | 41 |
| 7, 82 | 11, 3 | 22 |
| 8,25 | 10,7 | 18 |
| 8,47 | 10,4 | 38 |
| 10,32 | 8,6 | 32 |
| 10,85 | 8,2 | 18 |
| 11,86 | 7,46 | 29 |
| 12,61 | 7,01 | 26 |
| 13,46 | 6,58 | 49 |
| 13,81 | 6,41 | 19 |
| 14,29 | 6,20 | 37 |
| 14,48 | 6,11 | 42 |
| 14,97 | 5,92 | 53 |
| 15,28 | 5,80 | 96 |
| 16,88 | 5,25 | 65 |
| 17,39 | 5,10 | 39 |
| 17,78 | 4,99 | 42 |
| 18,41 | 4,81 | 25 |
| 19,08 | 4,65 | 50 |
| 19,55 | 4,54 | 14 |
| 20,10 | 4,41 | 28 |
| 21,12 | 4,20 | 24 |
| 21,70 | 4,09 | 19 |
| 21,95 | 4,05 | 25 |
| 22,80 | 3,90 | 26 |
| 25,65 | 3,47 | 100 |
| 26, 02 | 3,42 | 43 |
| 27,04 | 3,30 | 35 |
| 27,37 | 3,26 | 26 |
| 28,29 | 3,15 | 19 |
| 28,92 | 3,09 | 14 |
| 30,43 | 2,94 | 13 |

Das Röntgen-Pulver Diffraktions-Pattern ist in Abbildung 6 wiedergegeben.

### 2.3 Thermische Analyse

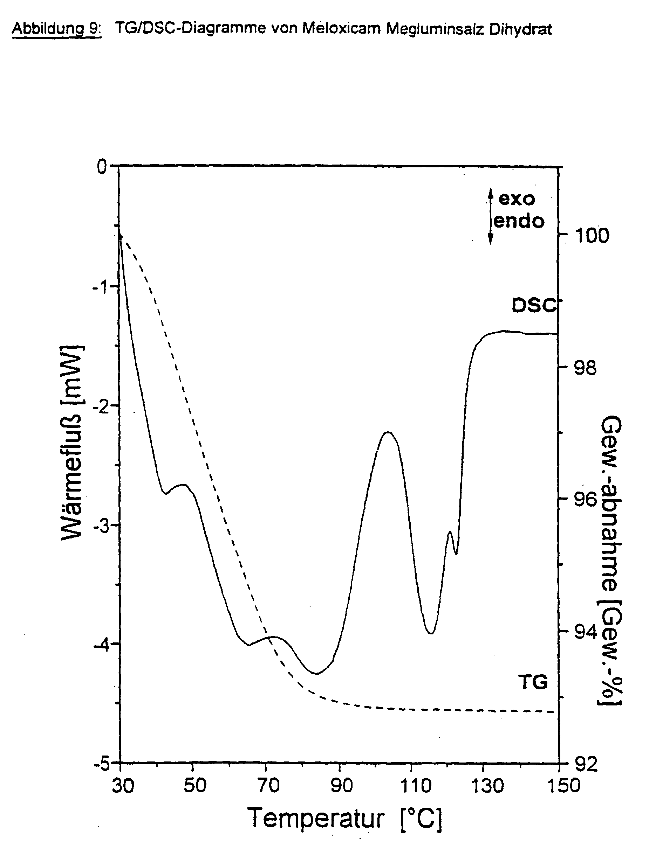

Eine klare Korrelation der im DSC-Diagramm beobachteten endothermen Peaks ist nicht möglich, da Dehydratisierungs- und Schmelzprozesse überlappen.
Die erhaltenen TG/DSC-Diagramme sind in Abbildung 8 und 9 wiedergegeben.
Das DSC Diagramm der Dihydratform ist sehr charakteristisch mit einem breiten und strukturierten endothemen Peak zwischen Raumtemperatur und 130 °C. Fünf klare Minima sind bei etwa 45, 65, 85, 115 und 125 °C sichtbar. Der Vergleich mit dem DSC-Diagramm der Monohydratform in Abbildung 8 zeigt klar die Differenzen zwischen diesen beiden Hydratformen. Gemeinsam sind lediglich die Endothermen bei etwa 85-90 °C (Dehydratisierungsschritt) und bei etwa 125 °C (Schmelzprozeß).

### Beispiel 3: Wasserfreies Meloxicam Megluminsalz

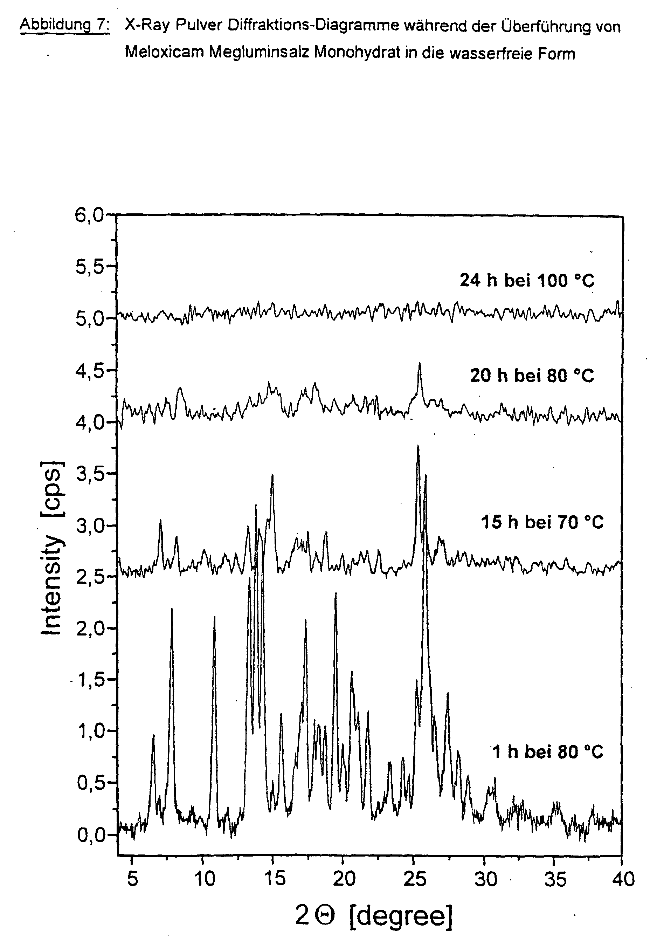

Meloxicam Megluminsalz Monohydrat kann durch Dehydratisierung in eine wasserfreie Form überführt werden. Die relevanten Parameter des Dehydratisierungsprozesses sind die Temperatur und die Dauer der Dehydratisierung, deren Einfluß durch Röntgen-Pulver Diffraktion beobachtet wurden. Je länger der Dehydratisierungsprozess andauert, umso weniger kristallin ist das resultierende Material. Nach 24 Stunden bei 100 °C ist das Meloxicam Megluminsalz wasserfrei und völlig amorph, während nach einer Stunde bei 80 °C keine Veränderung des eingesetzten Monohydrats festgestellt werden kann. Die nach 1 Stunde bei 80 °C, 15 Stunden bei 70 °C, 20 Stunden bei 80 °C und 24 Stunden bei 100 °C erhaltenen Röntgen-Pulver Diffraktions-Diagramme sind in Abbildung 7 wiedergegeben.

### Beispiel 4: Meloxicam Megluminsalz (Monohydrat) Tabletten, direktverpreßt

| | | |
|---|---|---|
| Rezeptur Meloxicam Megluminsalz Tabletten | Meloxicam Megluminsalz berechnet als Meloxicam | 7.5 mg |
| | Mikrokristalline Cellulose | 205.5 mg |
| | Laktose | 205.5 mg |
| | Polyvinylpyrrolidon (quervernetzt) | 22.5 mg |
| | Magnesiumstearat | 4.5 mg |

### Herstellung:

Die Wirksubstanz (gemahlen oder nicht gemahlen) wird mit den in der Rezeptur angegebenen Hilfsstoffen innig vermischt und direkt zu Tabletten verpreßt.

### Beispiel 5: Meloxicam Natriumsalz Tabletten, direktverpreßt

| | | |
|---|---|---|
| Rezeptur Meloxicam Natriumsalz Tabletten | Meloxicam Natriumsalz berechnet als Meloxicam | 7.5 mg |
| | Mikrokristalline Cellulose | 209.5 mg |
| | Laktose | 205.5 mg |
| | Polyvinylpyrrolidon (quervernetzt) | 22.5 mg |
| | Magnesiumstearat | 4.5 mg |

### Herstellungsverfahren:

Das Meloxicam Natriumsalz (gemahlen oder nicht gemahlen), hergestellt beispielsweise entsprechend den Angaben in der EP-A-0 002 482, wird mit den in der Rezeptur angegebenen Hilfsstoffen innig vermischt und direkt zu Tabletten verpreßt.

### Beispiel 6: in-vitro Freisetzung: Meloxicam (neutral)/direkt verpreßt versus Meloxicam (neutral)/granuliert/verpreßt

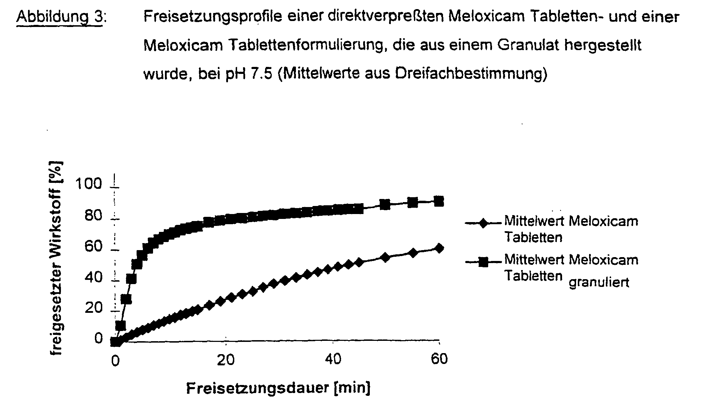

Wenn die Freisetzungsprofile zweier Tabletten miteinander verglichen werden, wobei die eine Formulierung durch Verpressen einer Pulvermischung, die andere durch Verpressen zuvor granulierten Pulvers hergestellt wurde, ist erkennbar, daß das Meloxicam aus der durch Verpressen der Pulvermischung hergestellten Tablette schneller freigesetzt wird (Abbildung 3). Die Freisetzung wurde über den Untersuchungszeitraum durch spektralphotometrische Bestimmung des Wirkstoffs bei dessen Extinktionsmaximum bestimmt.

| | | |
|---|---|---|
| Rezeptur Meloxicam Tabletten (direktverpreßt aus Pulver) | Meloxicam | 7.5 mg |
| | Mikrokristalline Cellulose | 210.0 mg |
| | Laktose | 205.0 mg |
| | Polyvinylpyrrolidon (quervernetzt) | 22.5 mg |
| | Magnesiumstearat | 4.5 mg |

| | | |
|---|---|---|
| Rezeptur Meloxicam Tabletten (verpreßt aus Granulat) | Meloxicam | 7.5 mg |
| | Mikrokristalline Cellulose | 210.0 mg |
| | Laktose | 205.0 mg |
| | Polyvinylpyrrolidon (quervernetzt) | 22.5 mg |
| | Magnesiumstearat | 4.5 mg |

### Beispiel 7: Humanversuche zur Verifizierung der Vorteile der erfindungsgemäßen Arzneiform gegenüber einer konventionellen Darreichungsform.

Die folgenden Formulierungen wurden an 18 Probanden als Einfachgabe im cross over untersucht:

| | | |
|---|---|---|
| Rezeptur Meloxicam Megluminsalz Tabletten (direktverpreßt) | Meloxicam Megluminsalz berechnet als Meloxicam | 7.5 mg |
| | Mikrokristalline Cellulose | 205.5 mg |
| | Laktose | 205.5 mg |
| | Polyvinylpyrrolidon (quervernetzt) | 22.5 mg |
| | Magnesiumstearat | 4.5 mg |

| | | |
|---|---|---|
| Rezeptur Meloxicam (granuliert) Kapseln | Meloxicam | 7.5 mg |
| | Natriumcitrat | 15.0 mg |
| | Mikrokristalline Cellulose | 102.0 mg |
| | Laktose | 23.5 mg |
| | Polyvinylpyrrolidon (löslich) | 10.5 mg |
| | Siliciumdioxid (hochdispers) | 3.5 mg |
| | Polyvinylpyrrolidon (quervernetzt) | 16.3 mg |
| | Magnesiumstearat | 1.7 mg |

In Abbildung 4 sind die Mittelwerte der erzielten Plasmaspiegel dargestellt. Es ist erkennbar, daß die im in vitro Dissolutionsverfahren festgestellten Unterschiede sich auch an den Blutspiegeln am Menschen nach peroraler Applikation zeigen. Bei Verwendung der schnell freisetzenden Form mit dem Salz des Meloxicams stiegen die Plamaspiegel schneller an, was zu einer Erhöhung der maximalen Plasmaspiegel sowie einer Verkürzung der Zeit, die zum Erreichen dieser Spiegel erforderlich ist, führte.

Bei einem Plasmaspiegel korrelierten Wirkungseintritt ist mit einer solchen Formulierung eine schneller einsetzende analgetische Wirkung zu erzielen.

## Patentansprüche

1. Oral applizierbare, feste Arzneimittelform von Meloxicam, aus der der Wirkstoff schnell freigesetzt und absorbiert wird, zur Schmerztherapie, **dadurch gekennzeichnet, daß** Meloxicam in Form eines Salzes mit einer anorganischen oder organischen Base, vorzugsweise des Natrium-, Kalium- oder Ammoniumsalzes, des Megluminsalzes, des Tris-Salzes oder des Salzes einer basischen Aminosäure, gegebenenfalls zusammen mit üblichen Hilfsstoffen oder/und Trägerstoffen, in einer schnell zerfallenden, durch Direkttablettierung hergestellten Tablette vorliegt.

2. Arzneimittelform gemäß Anspruch 1, **dadurch gekennzeichnet, daß** das Meloxicamsalz das Natrium- oder Megluminsalz ist.

3. Verfahren zur Herstellung einer oral applizierbaren, festen Arzneimittelform von Meloxicam gemäß Anspruch 1, **dadurch gekennzeichnet, daß** ein gegebenenfalls pulverisiertes Meloxicamsalz einer anorganischen oder organischen Base, vorzugsweise das Natrium-, Kalium- oder Ammoniumsalz, das Megluminsalz, das Tris-Salz oder das Salz einer basischen Aminosäure, zusammen mit üblichen pulverisierten Hilfsstoffen und / oder Trägerstoffen innig vermischt und ohne Granulation des Pulvers direkt zu Tabletten verpreßt wird.

4. Verfahren gemäß Anspruch 3, **dadurch gekennzeichnet, daß** das Meloxicamsalz das Natrium- oder Megluminsalz ist.

5. Kristallines Meloxicam-Megluminsalz Monohydrat und kristallines Meloxicam-Megluminsalz Dihydrat.

6. Verfahren zur Herstellung von kristallinem Meloxicam-Megluminsalz Monohydrat gemäß Anspruch 5, **dadurch gekennzeichnet, daß** Meloxicam und Meglumin in einer Mischung aus einem mit Wasser mischbaren organischen Lösungsmittel, vorzugsweise Aceton, Methanol, Ethanol, n-Propanol, i-Propanol, Tetrahydrofuran oder Dioxan, und Wasser erhitzt werden und die Mischung zur Kristallisation mit Meloxicam-Megluminsalz Monohydrat-Impfkristallen versetzt wird.

7. Verfahren gemäß Anspruch 6, **dadurch gekennzeichnet, daß** als organisches Lösungsmittel Aceton oder Ethanol verwendet wird.

8. Verfahren gemäß Anspruch 7, **dadurch gekennzeichnet, daß** in der Mischung organisches Lösungsmittel und Wasser im Volumenverhältnis von 10:1 bis 100:1 eingesetzt werden.

9. Verfahren gemäß Anspruch 6, **dadurch gekennzeichnet, daß** Meloxicam und Meglumin in einem molaren Verhältnis von 1:1,5 bis 1,5:1 eingesetzt werden.

10. Verfahren gemäß Anspruch 6, **dadurch gekennzeichnet, daß** bei einem Ansatz von A = 12,5 kg Meloxicam der Mischung B = 5 bis 50 g Meloxicam-Megluminsalz Monohydrat-Impfkristalle oder bei einer anderen Ansatzgröße Impfkristalle im entsprechenden Gew.-Verhältnis A : B = 125 : 0,05 - 0,5 zugefügt werden.

11. Verfahren zur Herstellung von kristallinem Meloxicam-Megluminsalz Dihydrat gemäß Anspruch 5, **dadurch gekennzeichnet, daß** kristallines Meloxicam-Megluminsalz Monohydrat bei hoher Luftfeuchtigkeit konditioniert wird, vorzugsweise durch Lagerung während mindestens eines Tages bei einer relativen Luftfeuchtigkeit von mindestens 75 %.

12. Oral applizierbare, feste Arzneimittelform gemäß Anspruch 1, enthaltend Meloxicam in Form des kristallinen Meloxicam-Megluminsalz Monohydrats.

13. Oral applizierbare, feste Arzneimittelform gemäß Anspruch 1, enthaltend Meloxicam in Form des kristallinen Meloxicam-Megluminsalz Dihydrats.

## Claims

1. Orally administered, solid pharmaceutical form of meloxicam, from which the active substance is rapidly released and absorbed, for pain therapy, **characterised in that** meloxicam is present in the form of a salt with an inorganic or organic base, preferably the sodium, potassium or ammonium salt, the meglumin salt, the Tris salt or the salt of a basic amino acid, optionally together with conventional excipients and/or carriers, in a rapidly decomposing tablet produced by direct tabletting.

2. Pharmaceutical form according to claim 1, **characterised in that** the meloxicam salt is the sodium or meglumin salt.

3. Process for preparing an orally administered solid pharmaceutical form of meloxicam according to claim 1, **characterised in that** an optionally pulverised meloxicam salt of an inorganic or organic base, preferably the sodium, potassium or ammonium salt, the meglumin salt, the Tris salt or the salt of a basic amino acid, is intimately mixed with conventional powdered excipients and/or carriers and compressed directly into tablets with no granulation of the powder.

4. Process according to claim 3, **characterised in that** the meloxicam salt is the sodium or meglumin salt.

5. Crystalline meloxicam meglumin salt monohydrate and crystalline meloxicam meglumin salt dihydrate.

6. Process for preparing crystalline meloxicam-meglumin salt monohydrate according to claim 5, **characterised in that** meloxicam and meglumin are heated in a mixture of a water-miscible organic solvent, preferably acetone, methanol, ethanol, n-propanol, i-propanol, tetrahydrofuran or dioxane, and water, and meloxicam-meglumin salt monohydrate seed crystals are added to the mixture for crystallisation.

7. Process according to claim 6, **characterised in that** acetone or ethanol is used as the organic solvent.

8. Process according to claim 7, **characterised in that** in the mixture organic solvent and water are used in a ratio by volume of 10:1 to 100:1.

9. Process according to claim 6, **characterised in that** meloxicam and meglumin are used in a molar ratio of 1:1.5 to 1.5:1.

10. Process according to claim 6, **characterised in that**, in the case of a quantity of A = 12.5 kg meloxicam, B = 5 to 50 g of meloxicam-meglumin salt monohydrate seed crystals are added to the mixture or, in the case of a different quantity of mixture, seed crystals are added in a corresponding ratio by weight of A : B = 125 : 0.05 - 0.5.

11. Process for preparing crystalline meloxicam-meglumin salt dihydrate according to claim 5, **characterised in that** crystalline meloxicam-meglumin salt monohydrate is conditioned under high humidity, preferably by storing it for at least one day at a relative humidity of at least 75 %.

12. Orally administered solid pharmaceutical form according to claim 1, containing meloxicam in the form of the crystalline meloxicam meglumin salt monohydrate.

13. Orally administered solid pharmaceutical form according to claim 1, containing meloxicam in the form of the crystalline meloxicam meglumin salt dihydrate.

## Revendications

1. Forme médicamenteuse solide, applicable par voie orale, de meloxicam, à partir de laquelle le principe actif est libéré et absorbé rapidement, pour la thérapie de la douleur, **caractérisée en ce que** le meloxicam est présent sous forme d'un sel avec une base inorganique ou organique, de préférence du sel de sodium, de potassium ou d'ammonium, du sel de méglumine, du sel de tris ou du sel d'un acide aminé basique, éventuellement avec des adjuvants et/ou supports habituels, dans un comprimé à désagrégation rapide produit par compression directe en comprimé.

2. Forme médicamenteuse selon la revendication 1 **caractérisée en ce que** le sel de meloxicam est le sel de sodium ou de méglumine.

3. Procédé de production d'une forme médicamenteuse solide, applicable par voie orale, de meloxicam selon la revendication 1 **caractérisé en ce qu'**un sel de meloxicam, éventuellement pulvérisé, et d'une base inorganique ou organique, de préférence le sel de sodium, de potassium ou d'ammonium, le sel de méglumine, le sel de tris ou le sel d'un acide aminé basique, est mélangé intimement avec des adjuvants et/ou supports pulvérisés habituels et compressé directement en comprimés sans granulation de la poudre.

4. Procédé selon la revendication 3 **caractérisé en ce que** le sel de meloxicam est le sel de sodium ou de méglumine.

5. Sel de meloxicam et de méglumine monohydraté cristallin et sel de meloxicam et de méglumine dihydraté cristallin.

6. Procédé de production de sel de meloxicam et de méglumine monohydraté cristallin selon la revendication 5 **caractérisé en ce que** le meloxicam et la méglumine sont chauffés dans un mélange d'un solvant organique miscible à l'eau, de préférence l'acétone, le méthanol, l'éthanol, le n-propanol, le i-propanol, le tétrahydrofurane ou le dioxane, et d'eau et le mélange est additionné de germes cristallins de sel de meloxicam et de méglumine monohydraté pour la cristallisation.

7. Procédé selon la revendication 6 **caractérisé en ce que** l'on utilise l'acétone ou l'éthanol comme solvant organique.

8. Procédé selon la revendication 7 **caractérisé en ce que**, dans le mélange, le solvant organique et l'eau sont utilisés dans le rapport volumique de 10:1 à 100:1.

9. Procédé selon la revendication 6 **caractérisé en ce que** le meloxicam et la méglumine sont utilisés dans un rapport molaire de 1:1,5 à 1,5:1.

10. Procédé selon la revendication 6 **caractérisé en ce que**, pour une charge de A = 12, 5 kg de meloxicam du mélange on ajoute B = 5 à 50 g de germes cristallins de sel de meloxicam et de méglumine monohydraté, ou pour une autre taille de charge on ajoute des germes cristallins dans le rapport massique A:B = 125:0,05-0,5 correspondant.

11. Procédé de production de sel de meloxicam et de méglumine dihydraté cristallin selon la revendication 5 **caractérisé en ce que** le sel de meloxicam et de méglumine monohydraté cristallin est conditionné à une grande humidité de l'air, de préférence par stockage pendant au moins un jour à une humidité de l'air relative d'au moins 75 %.

12. Forme médicamenteuse solide, applicable par voie orale, selon la revendication 1 contenant du meloxicam sous forme du sel de meloxicam et de méglumine monohydraté cristallin.

13. Forme médicamenteuse solide, applicable par voie orale, selon la revendication 1 contenant du meloxicam sous forme du sel de meloxicam et de méglumine dihydraté cristallin.
